# EUROPEAN PATENT APPLICATION

(11) **EP 4 822 030 A1**
(43) Date of publication of application: **14.10.2026**
(21) Application number: 25169414.7
(22) Date of filing: 09.04.2025
(51) Int. Cl.: C12P 39/00, C12P 7/46, C12P 13/00

(54) **DEPLETION OF OXYGEN IN EXHAUST GASES BY MICROORGANISMS**

(71) Applicant: SCHWENK Zement GmbH & Co. KG, 89077 Ulm (DE)
(72) Inventor: Wendisch, Volker, 33617 Bielefeld (DE); Rogowski, Max, 33615 Bielefeld (DE); Junker, Nora, 33378 Rheda-Wiedenbrücker (DE); Takors, Ralf, 70565 Stuttgart-Vaihingen (DE); Notheisen, Jan, 71101 Schönaich (DE); Filbig, Melanie, 70653 Stuttgart (DE); Schroeder, Kerstin, 52076 Aachen (DE); Öztürk, Merve, 89075 Ulm (DE)
(74) Representative: Müller-Boré & Partner Patentanwälte PartG mbB

(57) **Abstract**

The present invention relates to a method for the depletion of oxygen (O₂) in an exhaust gas containing O₂ and CO₂, said method comprising a step of aerobic fermentation by introducing said exhaust gas into a bioreactor containing a culture of one or more types of aerobic microorganisms in a liquid culture medium.

## Description

The present invention relates to a method for the depletion of oxygen (O₂) in an exhaust gas containing O₂ and CO₂, said method comprising a step of aerobic fermentation by introducing said exhaust gas into a bioreactor containing a culture of one or more types of aerobic microorganisms in a liquid culture medium.

In order to establish a circular bioeconomy, it is necessary to reduce the use of fossil resources and the associated CO₂ emissions (greenhouse gas) into the atmosphere. Sustainable technologies should therefore replace fossil resources and CO₂ emissions in general should be greatly reduced.

In this context, it is also important that mineral-bound carbon that is e.g., released during the clinker process in cement production is not released into the atmosphere in the form of CO₂. Instead, it is very important in the interest of sustainability to introduce CO₂ into CO₂ conversion processes on a long-term basis, e.g., by converting these large quantities of CO₂ into recyclable materials through the use of biotechnological processes. At the same time, this also reduces the amount of otherwise released greenhouse gases of fossil origin (crude oil, natural gas) that would have been used as an alternative for the production of these valuable materials according to the state of the art.

Microorganisms, in particular CO₂-consuming microbes, in particular acetogens, are able to convert CO₂ as a sole carbon source from exhaust gas (e.g., from cement or lime production) into valuable substances such as organic acids and alcohols. In addition to CO₂, this also requires an electron donor, such as hydrogen (H₂). Irrespective of whether CO is co-consumed or not, this property of using CO₂ as a sole carbon source is defined herein as carboxydotrophic growth, and microorganisms having this property are defined herein as carboxydotrophic microorganisms. In principle, there are both aerobic and anaerobic carboxydotrophic microorganisms. For technical applications, the anaerobic application, i.e., the cultivation of these microorganisms in the absence of oxygen, is particularly preferable in terms of safety, as this avoids the simultaneous use of oxygen and hydrogen.

In order for these anaerobic microorganisms to be used successfully for the valorization of CO₂-containing exhaust gas streams, it is therefore necessary that the exhaust gas to be used no longer contains residual oxygen. However, production (manufacturing) processes including combustion processes such as, e.g., those that occur in the cement industry, typically end with overall residual oxygen contents in the range of about 5 to 18% (v/v), in some applications even higher. As these O₂ contents are too high for the above-mentioned microorganisms, the oxygen must be removed from an exhaust gas stream before microbial conversion.

Current technologies in this respect are in particular aimed at drastically increasing the concentration of CO₂ and the CO₂ content in the exhaust gas in order to ultimately use the CO₂ not only for carbon capture and utilization (CCU), but also for enabling carbon capture and storage (CCS), that is, transfer of the CO₂ to storage sites. Possible technical approaches include chemical absorption with amines, carbonate looping or oxyfuel combustion.

However, current solutions for CO₂ enrichment and oxygen depletion all have the intrinsic disadvantage that they are technically very complex and economically very costly to implement.

Accordingly, the technical problem underlying the present invention is the provision of simple and cost-efficient means for the depletion of oxygen in exhaust gases, in order to facilitate subsequent uses of the depleted exhaust gases in anaerobic biotechnological processes such as the microbial valorization of CO₂.

The solution to the above technical problem is achieved by the embodiments characterized in the claims.

In particular, the present invention relates to a method for the depletion of oxygen (O₂) in an exhaust gas containing O₂ and CO₂, said method comprising a step of aerobic fermentation by introducing said exhaust gas into a bioreactor containing a culture of one or more types of aerobically growing microorganisms in a liquid culture medium.

These aerobically growing microorganisms grow heterotrophically despite the complex CO₂ containing exhaust gas composition that is injected as oxygen source. Notably, the oxygen depleted tail gas of this aerobic stage can be well used as inlet gas of a subsequent anaerobic stage that contains microorganisms in a liquid culture medium.

In this context, the term "depletion of oxygen" relates to a reduction of the oxygen content in the exhaust gas such that low dissolved oxygen levels, microaerophilic or even strictly anaerobic cultivation conditions may be installed with the exhaust gas in subsequent cultivations. In specific embodiments, the above term relates to a reduction of the oxygen content in the exhaust gas of at least 0.1% (v/v), at least 1% (v/v), at least 5% (v/v), at least 10% (v/v), at least 15% (v/v), at least 20% (v/v), at least 25% (v/v), at least 30% (v/v), at least 35% (v/v), at least 40% (v/v), at least 45% (v/v), at least 50% (v/v), at least 55% (v/v), at least 60% (v/v), at least 65% (v/v), at least 70% (v/v), at least 75% (v/v), at least 80% (v/v), at least 82% (v/v), at least 84% (v/v), at least 86% (v/v), at least 88% (v/v), at least 90% (v/v), at least 91% (v/v), at least 92% (v/v), at least 93% (v/v), at least 94% (v/v), at least 95% (v/v), at least 95.5% (v/v), at least 96% (v/v), at least 96.5% (v/v), at least 97% (v/v), at least 97.5% (v/v), at least 98% (v/v), at least 98.5% (v/v), at least 99% (v/v), at least 99.2% (v/v), at least 99.4% (v/v), at least 99.5% (v/v), at least 99.6% (v/v), at least 99.7% (v/v), at least 99.8% (v/v), at least 99.9% (v/v), or 100% (v/v). In this context, it is preferred that the reduction of the oxygen in the exhaust gas is at least 10% (v/v), at least 30% (v/v), at least 50% (v/v), at least 75% (v/v), or at least 90% (v/v).

The term "exhaust gas containing O₂ and CO₂" is not particularly limited and refers to any exhaust gas, *e.g.,* industrial exhaust gas, containing O₂ and CO₂. This includes, *e.g.,* exhaust gases deriving from combustion processes, *e.g.,* using oil, gas, coal, coke, and/or alternative fuels from waste and/or biomass. In specific embodiments, the exhaust gas contains about 0.1 to 21% (v/v) O₂, preferably about 1 to about 20% (v/v) O₂, more preferably about 2 to about 18% (v/v) O₂, more preferably about 5 to about 15% (v/v) O₂, more preferably about 8 to about 12% (v/v) O₂, more preferably about 10 to about 12% (v/v) O₂, more preferably about 11 to about 12% (v/v) O₂, *e.g*., about 11% (v/v) O₂ or about 12% (v/v) O₂. Further, in specific embodiments, the exhaust gas contains about 5 to about 30% (v/v) CO₂, preferably about 5 to about 25% (v/v) CO₂, more preferably about 10 to about 20% (v/v) CO₂, more preferably about 13 to about 20% (v/v) CO₂, more preferably about 13 to about 18% (v/v) CO₂, e.g., about 13% (v/v) CO₂, about 14% (v/v) CO₂, about 15% (v/v) CO₂, about 16% (v/v) CO₂, about 17% (v/v) CO₂, about 18% (v/v) CO₂. Of note, all of the above contents of O₂ and CO₂ are given in relation to dry gas under standard conditions, and refer to daily average values. In this context, it should be noted that short-term fluctuations can exceed the ranges mentioned above. Further, the term "standard conditions" as used herein refers to a pressure of 1013 hPa and a temperature of 273 K.

In preferred embodiments, the exhaust gas further contains one or more of NOₓ (*i.e.*, NO and/or NO₂), CO, NH₃, and SO₂. In specific embodiments, the exhaust gas contains NOₓ in an amount of about 10 to about 1000 mg/Nm³, preferably about 20 to about 800 mg/Nm³, more preferably about 50 to about 600 mg/Nm³, more preferably about 50 to about 500 mg/Nm³, more preferably about 100 to about 400 mg/Nm³, more preferably about 100 to about 300 mg/Nm³, more preferably about 150 to about 250 mg/Nm³, more preferably about 180 to about 220 mg/Nm³, *e.g.,* about 200 mg/Nm³. Further, in specific embodiments, the exhaust gas contains CO in an amount of about 150 to about 1200 mg/Nm³, preferably about 200 to about 1200 mg/Nm³, more preferably about 200 to about 1000 mg/Nm³, more preferably about 200 to about 800 mg/Nm³, more preferably about 200 to about 600 mg/Nm³, more preferably about 200 to about 400 mg/Nm³, more preferably about 200 to about 300 mg/Nm³, e.g., about 250 mg/Nm³. Of note, all of the above contents of NOₓ and CO are given are given in relation to 1 m³ of dry gas under standard conditions with an oxygen content of 10% (v/v), and refer to daily average values. In this context, it should be noted that short-term fluctuations can exceed the ranges mentioned above.

Further, typical contents of the further components of the exhaust gas defined above can be as follows. NH₃ in an amount of 0 to about 70 mg/Nm³, preferably about 0 to about 50 mg/Nm³, more preferably about 0 to about 30 mg/Nm³, more preferably about 5 to about 25 mg/Nm³ (e.g., about 25 mg/Nm³), more preferably about 10 to about 20 mg/Nm³ (e.g., about 20 mg/Nm³), more preferably about 13 to about 17 mg/Nm³, e.g., about 15 mg/Nm³. Furthermore, SO₂ can be present in the exhaust gas in an amount of 0 to about 300 mg/Nm³, preferably 0 to about 200 mg/Nm³, more preferably 0 to about 100 mg/Nm³, more preferably 0 to about 50 mg/Nm³, more preferably 0 to about 20 mg/Nm³, more preferably 0 to about 10 mg/Nm3, more preferably 0 to about 5 mg/Nm³, e.g., about 2.5 mg/Nm³. All of the above contents of NH₃ and SO₂ are given in relation to 1 m³ of dry gas under standard conditions with an oxygen content of 10% (v/v), and refer to daily average values. In this context, it should be noted that short-term fluctuations can exceed the ranges mentioned above.

Moreover, the exhaust gas can contain total C (elemental carbon and carbon-containing compounds; *i.e.,* total organic carbon (TOC)) in an amount of 0 to about 50 mg/Nm³, preferably 0 to about 25 mg/Nm³, more preferably 0 to about 15 mg/Nm³, *e.g.,* about 6, 7, 8, 9, or 10 mg/Nm³. The exhaust gas can further contain Hg in an amount of about 1 to about 40 µg/Nm³, preferably about 1 to about 20 µg/Nm³, more preferably 2 to about 10 µg/Nm³ (*e.g.,* about 10 µg/Nm³), more preferably 4 to about 8 µg/Nm³, *e.g.,* about 6 µg/Nm³. Moreover, the exhaust gas can contain residual dust in an amount of 0 to about 8 mg/Nm³, preferably 0 to about 5 mg/Nm³, more preferably 0 to about 2 mg/Nm³, more preferably 0 to about 1 mg/Nm³, *e.g.,* about 0.5 mg/Nm³. All of the above contents of TOC, Hg, and residual dust are given in relation to 1 m³ of dry gas under standard conditions with an oxygen content of 10% (v/v), and refer to daily average values. In this context, it should be noted that short-term fluctuations can exceed the ranges mentioned above.

In specific embodiments, the exhaust gas further contains one or more of HCl, HF, benzol, Cd, Tl, Sb, As, Pb, Cr, Co, Cu, Mn, Ni, V, Sn, benzo(a)pyrene, polychlorinated dibenzodioxins (PCDD), and polychlorinated dibenzofurans (PCDF). Specifically, HCl can be present in an amount of 0 to about 8 mg/Nm³, HF in an amount of 0 to about 1 mg/Nm³, benzol in an amount of 0 to about 3 mg/Nm³, Cd and Tl, taken together, in an amount of 0 to about 0.008 mg/Nm³, Sb, As, Pb, Cr, Co, Cu, Mn, Ni, V, and Sn, taken together, in an amount of 0 to about 0.2 mg/Nm³, As, benzo(a)pyrene, Cd, Co, and Cr, taken together, in an amount of 0 to 0.1 mg/Nm³, and PCDD and PCDF, taken together, in an amount of 0 to about 0.08 ng/Nm³. All of the above contents are given in relation to 1 m³ of dry gas under standard conditions with an oxygen content of 10% (v/v), and refer to average values. In this context, it should be noted that short-term fluctuations can exceed the ranges mentioned above.

In specific embodiments, the exhaust gas is exhaust gas derived from cement production, *e.g.,* taken from a cement production kiln tail end or extracted from the process. The kiln may be equipped with a calciner or precalciner firing with or without tertiary air duct. The fuel for firing the kiln and its calciner/precalciner might range from pure substances to mixtures/blends of any kind of, *e.g.,* fossil sourced, fuel such as but not limited to various oils, coal and lignite, waste, alternative fuel, refuse derived fuel (RDF), used tires, wood/timber, biomass/organic substances, natural gas, biogas, methanol, methane, hydrogen and its chemical and biological derivatives, sewage sludge, dried sewage sludge (both of which can be derived from municipal sewage treatment plants, industrial sewage treatment plants, or biological sewage treatment plants) and other fuels. For combustion, ambient air as well as oxygen or a mixture of air and oxygen may be used. The process may be equipped with a gas bypass for extraction of impurities e.g., chlorine-, sodium-, potassium- and sulfur-laden gas and/or dust, a SCR unit (Selective Catalyzed Reduction unit) - for reduction of NOₓ and NH₃ and CO, a mercury reduction unit, a scrubber for various impurities, a post combustion unit for organic components, fabric or ceramic dust filters or electric precipitators, as well as gas cooling devices such as water evaporators and/or heat exchangers being used for cooling or heating or heat displacement. Furthermore, the exhaust gas from the burning process can be used to dry the raw meal in the raw mill, whereafter the exhaust gas is sent to the stack. The exhaust gas from the clinker cooler can also be emitted together with the exhaust gas from the clinker burning process in one stack.

In some embodiments, the exhaust gas can be cooled before introduction into the bioreactor, *e.g.,* by way of a heat exchanger. Preferable temperatures to which the exhaust gas is cooled are temperatures that are tolerable for the microorganisms used in the method, *e.g.,* temperatures of about 20 to about 40°C, preferably of about 20 to about 37°C.

Further, in some embodiments, the exhaust gas is subjected to gas scrubbing to remove impurities before being introduced into the bioreactor.

In preferred embodiments, the one or more types of aerobically growing microorganisms are able to use oxygen of the inlet gas as final electron acceptor while growing on heterotrophic carbon sources. More preferably, the heterotrophic carbon sources are in the liquid culture medium and are the liquid products of the anaerobic stages that use the tail gas of the aerobic stage. Respective means for the culture of aerobic microorganisms, including the production culture for the production of a target product, are not particularly limited and are known in the art.

In respective embodiments, the method preferably further comprises, in addition to the step of aerobic fermentation, a step of anaerobic fermentation in a further bioreactor containing anaerobic microorganisms, in particular CO₂-consuming microbes, in particular acetogens, that anaerobically produce a product, wherein said product is recycled to the bioreactor containing the culture of one or more types of aerobic microorganisms, and wherein said product is used as said substrate for microbial growth and product formation. Respective means for the culture of anaerobic microorganisms, including the production culture for the production of a substrate product, are not particularly limited and are known in the art. Further, means for recycling the product to be used as substrate product to another bioreactor are not particularly limited and are known in the art.

Preferably, respective methods further comprise a step of recycling at least a part of a tail gas obtained from the aerobic gas fermentation to the bioreactor containing the culture of one or more types of anaerobic microorganisms, in particular CO₂ consuming microbes, in particular acetogens. In this context, the term "tail gas" refers to gas that is obtained as a byproduct of the fermentation, *i.e.,* a gas that, by way of the fermentation, is depleted in O₂ and enriched in CO₂, which can be easily recovered from the bioreactor in which the fermentation takes place. Respective means of recycling at least a part of said tail gas to another bioreactor are not particularly limited and are known in the art. In specific embodiments, H₂ is added to said tail gas prior to recycling the same to the other bioreactor.

The aerobically growing microorganisms used in the methods of the present invention are preferably selected from the group consisting of *Escherichia coli* and *Corynebacterium glutamicum.* In this respect, *E. coli* RM214 is an exemplary strain of *E. coli,* and *C. glutamicum* ATCC13032 and *C. glutamicum* GSLA *pECXT99A-ldcC pEK0-pck* (*C. glutamicum* GSLA *IdcC pck* hereinafter) are preferred strains of *C*. *glutamicum.* In this context, *C. glutamicum* can also switch to nitrate compounds at low oxygen levels in order to reduce the oxygen content even further.

The anaerobic microorganisms are preferably selected from the group of CO₂ fixing bacteria, preferably acetogens, more preferably selected from the group consisting of *Acetobacterium woodii, Acetobacterium wieringae, Clostridium ljungdahlii, Moorella thermoacetica,* and *Clostridium autoethanogenum.*

The tail gas obtained from aerobic fermentation is, by way of the fermentation, depleted of O₂ and enriched in CO₂. Preferably, said tail gas contains less than about 1% (v/v) O₂, preferably less than about 0.3% (v/v) O₂ and about 10% (v/v) to about 30% (v/v) CO₂.

In preferred embodiments, the methods of the present invention further comprise a step of recovering the target product from the aerobic culture. Means for recovering the target product from said culture are not particularly limited and are known in the art.

In specific embodiments, the product produced by the anaerobic microorganisms, i.e., the product to be used as the substrate product in the aerobic fermentation, is preferably selected from the group of products derived from the metabolism of acetogens, more preferably from the group consisting of organic acids and alcohols, such as formic acid, acetic acid, butyric acid, isobutyric acid, ethanol, isobutanol, hexanol, and the like.

Further, in specific embodiments, the target product produced in the aerobic fermentation is selected from the group consisting of organic acids or derivatives thereof, amino acids or derivatives thereof (*e.g.,* cadaverine), alcohols or derivatives thereof, proteins, lipids, or derivatives thereof, and biomass. In a preferred embodiment, monomers such as dicarboxylic acids, amino acids and diamines are produced that can be applied to produce plastics such as polypropylene, polyethylene, polyamides, and the like.

As used herein, the term "comprising"/"comprises" expressly includes the terms "consisting essentially of"/"consists essentially of" and "consisting of"/"consists of", *i.e.,* all of said terms are interchangeable with each other herein.

Further, as used herein, the term "about" refers to a modifier of the specified value of ± 10%, preferably ± 9%, ± 8%, ± 7%, ± 6%, ± 5%, ± 4%, ± 3%, ± 2%, ± 1%, ± 0.5%, or ± 0.1%. Thus, by way of example the term "about 100" as used herein can refer to ranges of 90 to 110, 91 to 109, 92 to 108, 93 to 107, 94 to 106, 95 to 105, 96 to 104, 97 to 103, 98 to 102, 99 to 101, 99.5 to 100.5, or 99.9 to 100.1.

The present invention focuses on the use of microorganisms for oxygen depletion in real exhaust gas streams, in order to subsequently enable the utilization of the exhaust gas stream for the microbial valorization of the CO₂.

Specifically, the invention is based on a multiple-stage cascade process, preferably a two-stage process. In a first anaerobic stage, a product to be metabolized, *e.g.,* acetate, is to be produced with the aid of *e.g.,* acetogenic microorganisms. The product enters the second stage operating under aerobic conditions where it serves as carbon source for the production of the product of interest in the presence of oxygen. Either the product formation itself or coinciding biological reactions consume oxygen. The product formation may target the production of monomers for subsequent polymerization, for example.

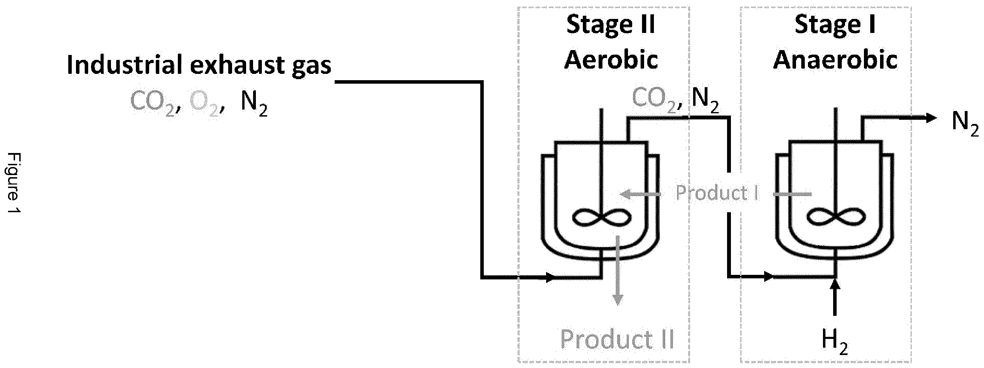

The core of the present invention lies in the fact that the second stage, which is operated in the presence of oxygen, is already directly gassed with the exhaust gas from, for example, a conventional kiln in the cement industry. A scrubber could also be installed to eliminate impurities from the gas. The exhaust gas containing CO₂ and O₂ in this way is fed directly into an aerobic production process, for example for the cultivation of *Escherichia coli* or *Corynebacterium glutamicum* strains. The microbial metabolic performance of the cells reduces the oxygen content in the exhaust gas so that it can then be fed into the anaerobic first stage in an oxygen-depleted and CO₂-enriched state (Fig. 1).

The surprising and unexpected discovery during the investigation of the present invention was that the above-mentioned microorganisms, which are of great importance for industrial operations, can grow and produce in the presence of real exhaust gases. This drastically reduces the typical residual oxygen content of the exhaust gas and at the same time further enriches the CO₂ content through the metabolic performance of the cells. This means that the substrate (*e.g.,* acetate), which was produced in the first stage, is converted into biomass, product, and CO₂ in this (second) aerobic stage. The latter can be recycled into the first stage, which improves the carbon economy of the entire process and in turn reduces the emission of CO₂ into the atmosphere.

The utilization of microbial activities for the targeted reduction of the oxygen content from the exhaust gas of a cement kiln, as well as the utilization of these gases for the production of valuable substances through microbial activity and also for the enrichment of CO₂ in a cascaded process, is novel in the art.

Thus, the present invention makes it possible to feed oxygen-containing exhaust gases, for example from the kiln of a cement production plant, directly into a microbial utilization process, which means that the significantly more extensive investments for CO₂ enrichment and oxygen reduction of alternative technical solutions can advantageously be avoided.

As far as exhaust gases, *e.g.,* from cement production, can be introduced into algae cultures, these approaches differ fundamentally from the present invention, since algae are autotrophic organisms which, in the specified applications, essentially require the CO₂ of the exhaust gas for growth under phototrophic conditions. In contrast, the processes presented herein are based on the heterotrophic growth of microorganisms, in which the gassing by kiln exhaust gases acts as a substantial disturbance factor for the cells. The essential carbon compound is provided by organic compounds (*e.g.,* carbohydrates, organic acids). The invention presented herein therefore differs fundamentally from the described algae application.

The figures show:
Figure 1:
   Schematic representation of a two-stage process for the oxygen depletion of industrial exhaust gases with variable components of carbon dioxide, oxygen, and nitrogen, among others.
   In stage I, the emitted carbon dioxide is to be fixed microbially by anaerobic fermentation with microbes such as *Acetobacterium woodii.* The carbon is bound in biomass and organic acids or alcohols such as acetic acid or ethanol (product I). In a further stage II, product I is aerobically converted to product II with microorganisms such as *Escherichia coli* or *Corynebacterium glutamicum.* Possible products II can include succinate or 1,5-diaminopentane, which can be used as monomers for the production of polyamides. In the process, the industrial exhaust gas is first fed into stage II to remove the oxygen it contains. The low-oxygen exhaust gas from stage II can then be fed into anaerobic stage I for CO₂ fixation. Hydrogen must also be added for this purpose.
Figure 2:
   Growth rates µ for the cultivation of different bacterial strains in the presence of different gas atmospheres.
   The bacteria were cultivated in gas-tight sealed bottles with either synthetic exhaust gas or real exhaust gas from a cement kiln. The mean value and standard deviation of biological triplicates are shown.
Figure 3:
   Production kinetics for succinate with *E. coli* RM214 *ΔsdhAB ΔiclR* and 1,5-diaminopentane with *C. glutamicum* GSLA *ldcC pckA.*
   The bacteria were cultivated in gas-tight sealed bottles with either synthetic exhaust gas or exhaust gas from a cement kiln. Succinate and 1,5-diaminopentane (cadaverine) were quantified by liquid chromatography. The mean and standard deviation of biological triplicates are shown.
Figure 4:
   Oxygen concentration in the fermentation exhaust gas and biomass in the reactor for the cultivation of *E. coli* MG1655.
   The bacteria were cultivated in a bioreactor with ambient air. After the initial batch phase, an exponential feed was started in order to set a constant growth rate of µ=0.1 h⁻¹. During the feed phase, the bacteria were gassed with synthetic exhaust gas.

The present invention will be further illustrated by the following examples without being limited thereto.

### Examples

The following question was investigated experimentally to demonstrate the feasibility of the present invention.

### Gas tolerance:

Can microbial production strains such as wild types or genetically modified production strains of *Escherichia coli* or *Corynebacterium glutamicum* be cultivated in the presence of industrial exhaust gas?

### Example 1:

### Gas tolerance

The tolerance of production strains to kiln exhaust gas was tested. In addition to the main components nitrogen, carbon dioxide and oxygen, the kiln exhaust gas also contains very small amounts of nitrogen oxides, carbon monoxide, sulfur dioxide and other compounds that have a potentially inhibitory effect on microorganisms. The kiln exhaust gas was extracted from the chimney of a cement plant in Mergelstetten, Germany, and filled into gas bags. Synthetic exhaust gas was used as a reference in cultivation. This contains only carbon dioxide and oxygen in a nitrogen atmosphere. The gas mixture was created according to the annual average values for carbon dioxide and oxygen of the cement plant. The gas compositions are shown in Table 1.

**Table 1: Composition of gases used.**

| | Exhaust gas I | Exhaust gas II | Synthetic exhaust gas |
|---|---|---|---|
| CO₂ | 14.3% (v/v) | 15.3% (v/v) | 15.5% (v/v) |
| O₂ | 11.65% (v/v) | 11.23% (v/v) | 11.5% (v/v) |
| NOₓ | 197 mg/Nm³ | 193 mg/Nm³ | - |
| NH₃ | 26.8 mg/Nm³ | 10.4 mg/Nm³ | - |
| Total C | 4 mg/Nm³ | 3 mg/Nm³ | - |
| CO | 173 mg/Nm³ | 323 mg/Nm³ | - |
| Dust | 0.23 mg/Nm³ | 4.66 mg/Nm³ | - |
| SO₂ | 0.0245 mg/Nm³ | 5.94 mg/Nm³ | - |
| Hg | 6.3 µg/Nm³ | 9.15 µg/Nm³ | - |
| | in nitrogen | | |

### Experimental setup

The experiment was carried out in gas-tight sealed 1 L glass bottles with 30-50 mL culture volume and approx. 1 L headspace. The bottles were sealed with a butyl stopper and evacuated. The corresponding gas was then drawn into the bottle. For experiments with *C. glutamicum* strains and with the *E. coli* WT, kiln exhaust gas I was used. For the cultivation of *E. coli* RM214 *ΔsdhAB ΔiclR,* kiln exhaust gas II was used. The bottle was filled with the appropriate medium and then inoculated with an inoculum of the bacterial strain to be examined. Cultivation took place at 30 °C for *C. glutamicum* strains and 37 °C for *E. coli* strains with continuous shaking. The strains used can be found in Table 2. The composition of the M9 medium for *E. coli* is listed in Table 3 and the composition of the CgXII medium for *C. glutamicum* in Table 4. During cultivation, samples of the suspension were taken to determine the biomass concentration (turbidity measurement) and the concentration of any products produced in the culture supernatant using liquid chromatography.

**Table 2: Strains used.**

| Strain | Modifications |
|---|---|
| *Escherichia coli* MG1655 | wildtype |
| *Escherichia coli* RM214 *ΔsdhAB ΔiclR** | Deletion of succinate dehydrogenase and the regulator IcIR for succinate production |
| *Corynebacterium glutamicum* ATCC13032 | wildtype |
| *C. glutamicum* GSLA *pECXT99A-IdcC pEK0-pck* | Lysine producer with heterologous lysine decarboxylase for 1,5-diaminopentane production |

| | |
|---|---|
| * Ziegler M, *et al*.; Metab Eng. 2021 Sep; 67:75-87. doi: 10.1016/j.ymben.2021.05.011. PMID: 34098100 | |

**Table 3: M9 Medium for the cultivation of E. coli strains.**

| Component | Final concentration |
|---|---|
| NaH₂PO₄ 2 H₂O | 3.2 g/L |
| K₂HPO₄ | 11.7 g/L |
| (NH₄)₂SO₄ | 8 g/L |
| Thiamine-HCl | 0.01 g/L |
| Morpholinopropionic sulfonic acid | 50 g/L |
| Glucose | 20 g/L |
| FeCl₃ 6 H₂O | 8.35 mg/L |
| ZnSO₄ 7 H₂O | 0.09 mg/L |
| MnSO₄ H₂O | 0.05 mg/L |
| CuSO₄ 5 H₂O | 0.8 mg/L |
| CoCl₂ 2 H₂O | 4.4 mg/L |
| MgSO₄ 7 H₂O | 0.1 g/L |
| Trisodiumcitrate 2 H₂O | 0.11 g/L |

**Table 4: CgXII medium for the cultivation of C. glutamicum strains.**

| Component | Final concentration |
|---|---|
| (NH₄)₂SO₄ | 10 g/L |
| K₂HPO₄ | 1 g/L |
| KH₂PO₄ | 1 g/L |
| Urea | 5 g/L |
| Morpholinopropionic sulfonic acid | 21 g/L |
| Glucose | 20 g/L |
| FeSO₄ 2 H₂O | 8.35 mg/L |
| ZnSO₄ 7 H₂O | 0.09 mg/L |
| MnSO₄ H₂O | 0.05 mg/L |
| CuSO₄ 5 H₂O | 0.8 mg/L |
| NiCl₂ 6 H₂O | 4.4 mg/L |
| MgSO₄ 7H₂O | 0.25 g/L |
| CaCl₂ 2H₂O | 0.01 g/L |
| Biotin | 0.2 mg/L |
| 3,4-Dihydroxybenzoic acid | 30 mg/L |

The growth rate µ describes the biomass-specific increase in biomass over time and is defined as c_{X} = c_{X,0} e^{µ t}.

### Results

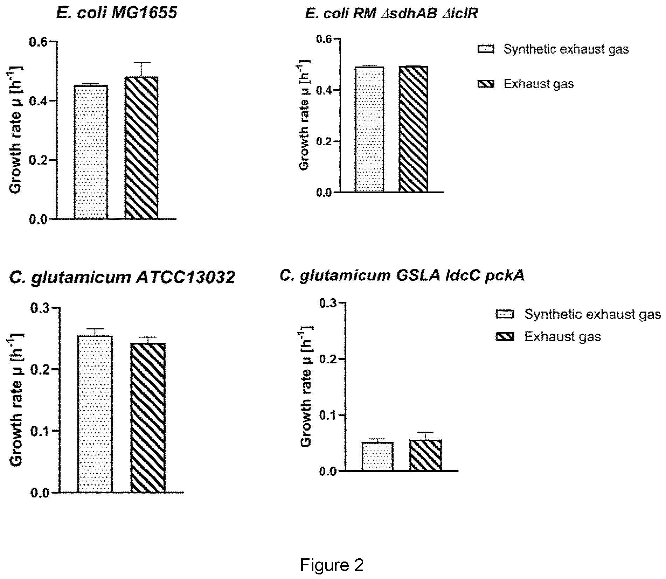

It can be seen that there is no difference in growth behavior between real and synthetic exhaust gas for any of the strains tested (Fig. 2). It can therefore be concluded that, surprisingly, the real exhaust gases from the kiln can be used to cultivate the cells and similar growth kinetics can be achieved as with simulated synthetic gases.

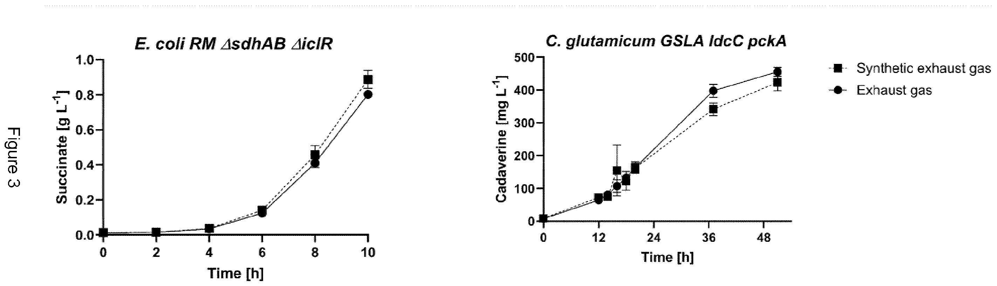

Looking at the production of the monomers succinate and 1,5-diamninopentane in the presence of real exhaust gas with the corresponding production strains, no difference between the synthetic exhaust gas and the kiln exhaust gas can be observed in the production kinetics, as was also the case with growth (Fig. 3). From this it can again be concluded that, surprisingly, the real exhaust gases from the kiln can be used and similar production kinetics can be achieved as with the synthetic gases.

### Example 2:

### Oxygen reduction

After demonstration of the cultivation and product formation in the presence of exhaust gas, the oxygen depletion by aerobic fermentation will now be shown. The kiln exhaust gas from the cement plant typically contains more than 10% (v/v) oxygen. For the cultivation of anaerobic microorganisms such as *Acetobacterium woodii,* an oxygen content of less than 1% (v/v) is typically required. *A. woodii* showed no growth at oxygen concentrations above 0.3 % while other acetogenic microorganisms showed growth at 1 % oxygen in the gas phase. Higher oxygen concentrations often have a toxic effect. In this case, aerobic fermentation (stage II, Figure 1) should help to reduce the oxygen content of the kiln exhaust gas through suitable process control so that it can be used further in the anaerobic process (stage I, Figure 1).

### Experimental setup

The experiments were carried out with the wild type *E. coli* MG1655 in minimal medium (Table 5). Cultivation took place in a 30 L stainless steel reactor at 37 °C, 2.5 L min⁻¹ gassing. After an initial batch phase, glucose was fed into the reactor as a carbon source at an exponential feed rate to realize a constant growth rate of µ=0.1 h⁻¹. During the batch phase, the reactor was gassed with ambient air. In the feed phase, gassing was switched to synthetic exhaust gas (Table 1) after the start of the feed.

**Table 5: M9 Medium for the cultivation of E. coli strains in the bioreactor.**

| Component | Final concentration |
|---|---|
| NaSO₄ | 2.0 g/L |
| K₂HPO₄ | 14.6 g/L |
| (NH₄)₂SO₄ | 8 g/L |
| Thiamine-HCl | 0.02 g/L |
| Glucose | 27.5 g/L |
| FeCl₃ 6 H₂O | 31.3 mg/L |
| ZnSO₄ 7 H₂O | 0.68 mg/L |
| MnSO₄ H₂O | 0.38 mg/L |
| CuSO₄ 5 H₂O | 0.6 mg/L |
| CoCl₂ 2 H₂O | 0.67 mg/L |
| MgSO₄ 7 H₂O | 0.5 g/L |
| Diammonium hydrogen citrate | 1.0 g/L |
| Ammonium chloride | 1 g/L |
| NaH₂PO₄ 2 H₂O | 4 g/L |
| Disodium-EDTA | 37.7 mg/L |
| CaCl₂ 2 H₂O | 1.9 mg/L |

### Results

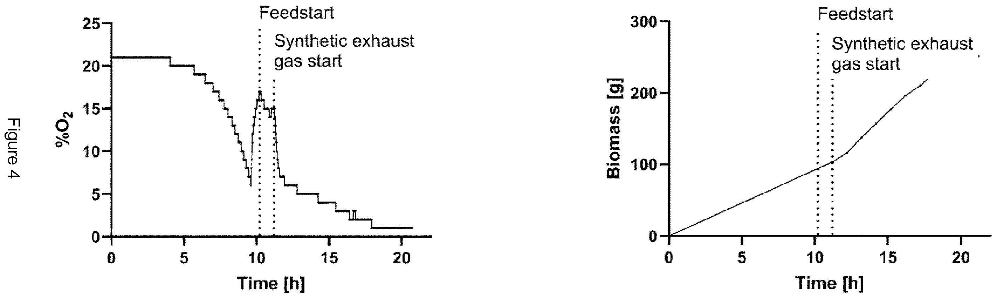

After the initial batch phase, exponential feeding of a highly concentrated glucose feed was started in order to set a constant growth rate of µ=0.1 h⁻¹. During the feed phase, gassing was switched to synthetic exhaust gas (Table 1). The oxygen content in the fermentation exhaust gas could be reduced to up to 1% (v/v) (Figure 4), while the biomass concentration continued to increase. Thus, it could be shown that 1) the cells grow in the presence of a gas atmosphere comparable to kiln exhaust gas from a cement plant and 2) the oxygen content of the gas can be significantly reduced by aerobic fermentation to reach concentrations suitable for anaerobic cultivation. Surprisingly, the high CO₂ content of over 15% (v/v) in the synthetic exhaust gas does not appear to disturb the aerobic bacterium *E. coli.* This is remarkable in view of the fact that these bacteria, in contrast to anaerobic microorganisms, are not designed to metabolize CO₂ and are therefore not naturally adapted to the presence of high CO₂ concentrations, as known in the art.

### Discussion:

In summary, it can be stated that the investigated microorganisms can grow in the presence of kiln exhaust gas and produce biotechnological products such as 1,5-diaminopentane and succinate. Surprisingly, no difference in growth and production kinetics were found in tests compared to synthetically adjusted exhaust gas. In addition, it was shown in the bioreactor that microorganisms can also be cultivated with continuous gassing with a gas mixture corresponding to the composition of a kiln exhaust gas (CO₂, O₂, N₂) and that the oxygen content of the gas can be reduced, so that the exhaust gas is theoretically suitable for the cultivation of anaerobic bacteria in stage I. At this point, another advantage of the process presented here should be pointed out. In addition to the oxygen reduction, the carbon dioxide content in the exhaust gas is increased in parallel by aerobic cultivation due to the metabolic activity of the cells. An increased carbon dioxide concentration in the gas enables better substrate availability in the anaerobic process.

## Claims

1. A method for the depletion of oxygen (O₂) in an exhaust gas containing O₂ and CO₂, said method comprising a step of aerobic fermentation by introducing said exhaust gas into a bioreactor containing a culture of one or more types of aerobic microorganisms in a liquid culture medium.

2. The method of claim 1, wherein the liquid culture medium comprises a substrate product, wherein the one or more types of aerobic microorganisms aerobically metabolize said substrate product and the exhaust gas to a target product.

3. The method of claim 2, further comprising, prior to the step of aerobic fermentation, a step of anaerobic fermentation in a bioreactor containing a culture of one or more types of anaerobic microorganisms in a liquid culture medium, wherein said one or more types of anaerobic microorganisms anaerobically produce a product, wherein said product is recycled to the bioreactor containing the culture of one or more types of aerobic microorganisms, and wherein said product is used as said substrate product that is metabolized to the target product.

4. The method of claim 3, further comprising a step of recycling at least a part of a tail gas obtained from the aerobic fermentation to the bioreactor containing the culture of one or more types of anaerobic microorganisms.

5. The method of any one of claims 1 to 4, wherein the aerobic microorganisms are selected from the group consisting of *Escherichia coli* and *Corynebacterium glutamicum.*

6. The method of claim 5, wherein the *C. glutamicum* is *C. glutamicum* ATCC13032 or *C. glutamicum* GSLA *pECXT99A-IdcC pEK0-pck.*

7. The method of any one of claims 3 to 6, wherein the anaerobic microorganisms are CO₂ fixing bacteria, preferably acetogens, preferably selected from the group consisting of *Acetobacterium woodii, Acetobacterium wieringae, Clostridium ljungdahlii, Moorella thermoacetica,* and *Clostridium autoethanogenum.*

8. The method of any one of claims 1 to 7, wherein the exhaust gas contains about 0.1 to about 21% (v/v) O₂, and about 5 to about 30% (v/v) CO₂, wherein said contents of O₂ and CO₂ are given in relation to dry gas under standard conditions, and refer to daily average values.

9. The method of any one of claims 1 to 8, wherein the exhaust gas further contains one or more of NOₓ (NO and/or NO₂), CO, NH₃, and SO₂.

10. The method of claim 9, wherein the exhaust gas contains NOₓ in an amount of about 50 to about 600 mg/Nm³, and/or CO in an amount of about 150 to about 1200 mg/Nm³, wherein said contents of NOₓ and CO are given in relation to 1 m³ of dry gas under standard conditions with an oxygen content of 10% (v/v), and refer to daily average values.

11. The method of any one of claims 1 to 10, wherein
(i) the exhaust gas is cooled before introduction into the bioreactor, and/or
(ii) the exhaust gas is subjected to gas scrubbing to remove coarse impurities before introduction into the bioreactor.

12. The method of any one of claims 1 to 11, wherein a tail gas obtained from the aerobic gas fermentation contains less than about 1% (v/v) O₂ and about 10% (v/v) to about 30% (v/v) CO₂, wherein said contents of O₂ and CO₂ are given in relation to dry gas under standard conditions, and refer to daily average values.

13. The method of any one of claims 1 to 12, further comprising the step of recovering the target product from the aerobic culture.

14. The method of any one of claims 1 to 13, wherein the substrate product is selected from the group consisting of organic acids and alcohols.

15. The method of any one of claims 1 to 14, wherein the target product is selected from the group consisting of organic acids or derivatives thereof, amino acids or derivatives thereof, alcohols or derivatives thereof, proteins, lipids, or derivatives thereof, and biomass.
